(19) Europäisches Patentamt
European Patent Office
Office européen des brevets

(11) Veröffentlichungsnummer: **0 355 025 A2**

(12) **EUROPÄISCHE PATENTANMELDUNG**

(21) Anmeldenummer: 89115003.9

(22) Anmeldetag: 14.08.89

(51) Int. Cl.⁴: C07C 43/225 , C07D 319/12 , C07C 47/575 , C07C 41/30 , C07C 41/06 , C07C 45/64 , C08F 12/22 , B01D 15/08 , C09J 125/18

(30) Priorität: 18.08.88 DE 3828063

(43) Veröffentlichungstag der Anmeldung: 21.02.90 Patentblatt 90/08

(84) Benannte Vertragsstaaten: BE DE FR GB IT NL

(71) Anmelder: **HOECHST AKTIENGESELLSCHAFT**
**Postfach 80 03 20**
**D-6230 Frankfurt am Main 80(DE)**

(72) Erfinder: **Fuss, Andreas, Dr.**
**Lindigstrasse 24**
**D-8757 Karlstein(DE)**
Erfinder: **Hintzer, Klaus, Dr.**
**Rupertusstrasse 3**
**D-8269 Burgkirchen(DE)**

(54) **Substituierte(2-Haloalkoxy-1.1.2-trifluoräthoxy)-styrole, Verfahren zu ihrer Herstellung und ihre Verwendung.**

(57) Styrole der allgemeinen Formel

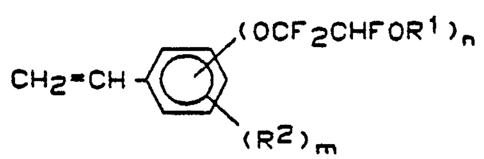

(I)

werden erhalten aus einem entsprechenden Hydroxyaldehyd, der mit mindestens n Mol eines Vinyläthers der Formel $CF_2=CFOR^1$ zu dem perfluorierten Aldehyd und anschließend mit einem Methylentriarylphosphoniumhalogenid, jeweils in Gegenwart von inerten, aprotischen Lösungsmittel sowie verschiedener Basen, zu Verbindungen (I) umgesetzt wird. Die Styrole (I) werden zur Herstellung von Polymeren verwendet, die als Trägermaterial für chromatographische Verfahren, als Material für optische Systeme, als Resists, als Formteile und Klebstoffe dienen.

EP 0 355 025 A2

## Substituierte(2-Haloalkoxy-1.1.2-trifluoräthoxy)-styrole, Verfahren zu ihrer Herstellung und ihre Verwendung

Die Erfindung betrifft neue (2-Haloalkoxy-1.1.2-trifluoräthoxy)-styrole, Verfahren zu ihrer Herstellung und Verwendung zur Herstellung von Polymeren.

Fluor-substituierte Styrole und Verfahren zu ihrer Herstellung sind bekannt (DE-OS 29 36 237). Allerdings beschreibt der Stand der Technik andere Produkte, auch das Verfahren weicht von dem der Erfindung ab. Das Verfahren der vorliegenden Erfindung zur Herstellung der neuen Flour-substituierten Styrole (I) geht von leichter zugänglichen Ausgangsmaterialien aus und benötigt weniger Verfahrensstufen.

Gegenstand der Erfindung sind neue (2-Haloalkoxy-1.1.2-trifluoräthoxy)-styrole der allgemeinen Formel

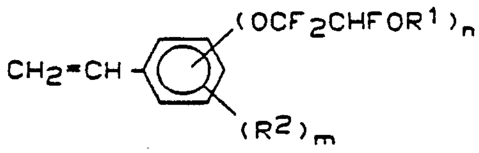

(I)

worin
$R^1$ eine teilweise oder vollständig halogenierte $C_1$-$C_{10}$-Alkyl-, vorzugsweise $C_1$-$C_3$-Alkylgruppe oder einen Rest der Formel

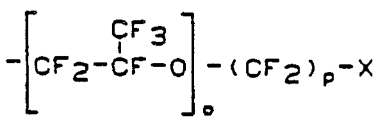

(II)

oder

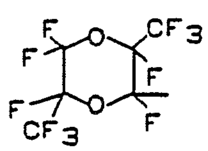

(III)

und $R^2$ Wasserstoff, Halogen oder eine teilweise oder vollständig halogenierte -$C_1$-$C_8$-, vorzugsweise $C_1$-$C_3$-Alkyl-Gruppe, wobei die Alkylgruppen $R^1$ und $R^2$ gleich oder verschieden sind,
m ist als ganze Zahl 1, 2, 3 oder 4,
n ist als ganze Zahl 1, 2 oder 3,
o ist als ganze Zahl 1, 2, 3 oder 4, p ist als ganze Zahl 1, 2, 3, 4, 5, 6, 7, 8, 9 oder 10 und X Halogen oder Wasserstoff mit der Maßgabe, daß die Summe aus m plus n nicht größer als 5 ist. Halogen ist Fluor, Chlor oder Brom, vorzugsweise jedoch Fluor.

Beispiele für $C_1$-$C_8$-Alkyl sind teilweise oder völlig halogeniertes, vorzugsweise fluoriertes Methyl, Äthyl, die verschiedenen Propyl- und Butyl-Gruppen. Pentyl, Hexyl, Octyl und Decyl, vorzugsweise Methyl, Äthyl und Propyl, insbesondere Äthyl und Propyl.

Ein weiterer Gegenstand der Erfindung ist ein Verfahren zur Herstellung der Styrole der allgemeinen Formel (I) in zwei Stufen (Variante A), bei dem

a) ein Hydroxybenzaldehyd der Formel

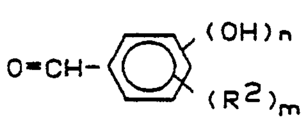

(IV)

mit mindestens n Mol eines Vinyläthers der allgemeinen Formel
$CF_2 = CFOR^1$ (V)
in Gegenwart einer Base $B^1$ zu einem Aldehyd der Formel

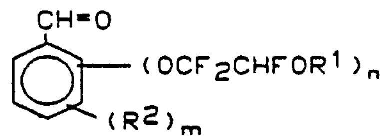

(VI)

umgesetzt wird,
wobei $R^1$, $R^2$, m und n die obengenannte Bedeutung besitzen und b) der gebildete Aldehyd (VI) mit Methyltriarylphosphoniumhalogenid in Gegenwart einer Base $B^2$ zu den Styrolen der Formel (I) umgesetzt werden.

Als Base $B^1$ können Hydroxide oder Carbonate von Lithium, Natrium, Kalium, Cäsium, Ammonium, vorzugsweise jedoch Kaliumcarbonat, Natrium- und Kaliumacetat, Lithium-, Natrium- und Kaliumfluorid, NaH, $NaNH_2$;
Kaliumtertiärbutylat oder Trialkylamin mit 1 bis 4 C-Atomen in der Alkylgruppe eingesetzt werden.

Die Base $B^2$ ist beispielsweise NaH, Kaliumtertiärbutylat, $NaNH_2$, $C_1$ bis $C_4$-Alkyllithium, Aryllithium mit 6 bis 12 C-Atomen in der Arylgruppe sowie Natriumalkoxid mit 1 bis 4 C-Atomen, vorzugsweise $NaNH_2$.

In einer anderen Verfahrensausführung (Variante B) wird das Acetoxystyrol der allgemeinen Formel

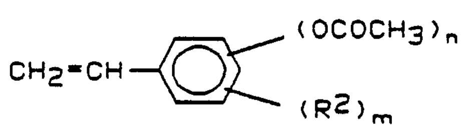

(VII)

worin $R^2$, m und n die obengenannte Bedeutung besitzen, zunächst mit - gegebenenfalls wässrigem - Alkalihydroxid $B^3$ und dann mit dem Vinyläther (V) zu den Styrolen (I) umgesetzt.

Beipiele für Styrole der Formel (I) sind:
o-[(2-Trifluormethoxy)-1.1.2-trifluoräthoxy]-styrol,
m-[(2-Trifluormethoxy)-1.1.2-trifluoräthoxy]-styrol,
p-[(2-Trifluormethoxy)-1.1.2 trifluoräthoxy]-styrol,
o-[(2-Pentafluoräthoxy)-1.1.2-trifluoräthoxy]-styrol,
m-[(2-Pentafluoräthoxy)-1.1.2-trifluoräthoxa]-styrol,
p-[(2-Pentafluoräthoxy)-1.1.2-trifluoräthoxy]-styrol,
o-[(2-Heptafluorpropoxy)-1.1.2-trifluoräthoxy]-styrol,
m-[(2-Heptafluorpropoxy)-1.1.2-trifluoräthoxy]-styrol,
p-[(2-Heptafluorpropoxy)-1.1.2-trifluoräthoxy-styrol,
p-[2-(1.1.2.2.3.3.-Hexafluorpropoxy)-1.1.2-trifluoräthoxy]-styrol, p [(2-Perfluorpentoxy)-1.1.2-trifluoräthoxy]-styrol, p- (2-Perfluoroctoxy)-1.1.2-trifluoräthoxy]-styrol,
p-[(2-Chlordifluormethoxy)-1.1.2-trifluoräthoxy -styrol,
o,m,p-Tris[2-(heptafluorpropoxy)-1.1.2-trifluoräthoxy]-styrol,
o,o′,p-Tris[2(heptafluorpropoxy)-1.1.2-trifluoräthoxy]-styrol,
m,m′,p-Tris 2-(heptafluorpropoxy)-1.1.2-trifluoräthoxy]styrol und o-Fluor-p- (2-heptafluorpropoxy)-1.1.2-trifluoräthoxy]-styrol.

Die Aldehyde (IV) und Styrole (VII) sind entweder bekannt oder können nach bekannten Verfahren hergestellt werden.

Die Umsetzung der Hydroxyaldehyde (IV) zu den Haloalkoxyaldehyden der Formel (VI) wird im allgemeinen in Gegenwart eines inerten, aprotischen Lösungsmittels ($C^1$) wie Aceton, Methyläthylketon, Methylisobutylketon, Nitromethan, Acetonitril, Propionitril, Benzonitril, Dimethylformamid, Dimethylacetamid, Tetrahydrofuran, 1.4-Dioxan, Dimethoxyäthan, Diäthylenglykoldimethyläther (Diglyme), Tetraäthylenglykoldimethyläther (Tetraglyme), Dimethylsulfoxid, Dimethylsulfon, N-Methylpyrrolidon, Sulfolan oder HexamMethylphosphorsäuretriamid oder Gemischen davon, vorzugsweise in Acetonitril, bei Temperaturen von minus 100 bis 100 °C, vorzugsweise bei 0 bis 30 °C durchgeführt. Die Lösungsmittel sind im allgemeinen wasserfrei, können aber auch einen Wassergehalt von bis zu 10 Gew.-% aufweisen, ohne eine Beeinträchtigung der Reaktion zu bewirken. Im Verfahren gemäß der Erfindung kann der Vinyläther (V) und die Base $B^1$

in stöchiometrischen Mengen, im Über- oder Unterschuß, bezogen auf den Hydroxyaldehyd (IV), eingesetzt werden. Die Base $B^1$ wird vorzugsweise im Molverhältnis 1 : 1 bis 0,01 : 1 bezogen auf die Verbindung (IV) eingesetzt. Die Reaktion kann bei atmosphärischen Druck oder erhöhten Druck von bis zu 100 bar, vorzugsweise aber bei Normaldruck durchgeführt werden.

In der Variante (A) des Verfahrens gemäß der Erfindung wird der Hydroxyaldehyd (IV), die Base $B^1$ und das Lösungsmittel vorgelegt und der Vinyläther bei der oben angegebenen Temperatur unter Rühren zudosiert und solange gerührt, bis das Ausgangsmaterial (IV) im Dünnschichtchromatogramm nicht mehr nachweisbar ist.

Die Umsetzung des Haloalkoxyaldehyds (VI) mit Methyltriarylphosphoniumhalogenid und der Base $B^2$ wird in einem inerten, wasserfreien Lösungsmittels( $C^2$) wie Diäthyläther, Dimethoxyäthan, Diglyme, Tetraglyme, Tetrahydrofuran, Dimethylformamid oder Dimethylsulfoxid, vorzugsweise in Tetrahydrofuran bei Temperaturen zwischen minus 100 °C bis 100 °C, vorzugsweise bei minus 78 °C bis 30 °C durchgeführt. Vorteilhaft wird das Methyltriarylphosphoniumhalogenid in dem inerten Lösungsmittel vorgelegt und zunächst mit der Basis $B^2$ bei 0 bis 50 °C zum Methylentriarylphosphoran umgesetzt. Dann wird bei minus 100 bis minus 50 °C vorzugsweise bei minus 80 °C bis minus 70 °C der Haloalkoxyaldehyd (VI) zudosiert und die Temperatur auf 20-30 °C ansteigen gelassen. Das Methyltriarylphosphoniumhalogenid und die Base $B^2$ können, bezogen auf den Haloalkoxyaldehyd (VI), im Molverhältnis 1 : 1 bis 10 : 1 eingesetzt werden.

Die Variante (B), d. h. die Umsetzung des Acetoxystyrols (VII) mit Alkalihydroxid $B^3$ wird in einem inerten, aprotischen Lösungsmittel ($C^1$) vorzugsweise in Acetonitril, bei Temperaturen von minus 100 bis 100 °C, vorzugsweise bei minsu 10 bis 50 °C durchgeführt.

Als Alkalihydroxid $B^3$ kann wasserfreies Lithium-, Natrium-oder Kaliumhydroxid oder eine wässrige Lösung davon sowie Ammoniumhydroxid eingesetzt werden, vorzugsweise 20 bis 50 %ige Natron- oder Kalilauge.

Wenn das Acetoxystyrol (VII) dünnschichtchromatographisch nicht mehr nachzuweisen ist, wird der Vinyläther (V) bei Temperaturen von minus 100 bis 100, vorzugsweise bei 0 bis 75 °C, insbesondere bei 20-30 °C zudosiert. Die Reaktion kann bei 0 bis 100 bar durchgeführt werden, 0 bar wird bevorzugt.

Die Styrole der Formel (I) lassen sich als Monomere oder als Comonomere zusammen mit ungesättigten polymerisierbaren Verbindungen zu Polystyrolen polymerisieren (siehe die am selben Tag eingereichte deutsche Patentanmeldung, Titel "Polymerisate aus substituierten (2-Haloalkoxy-1.1.2-trifluoräthoxy)-styrolen,Verfahren zu ihrer Herstellung und ihre Verwendung" P 38 28 064.7). Diese Polystyrole können eingesetzt werden als Trägermaterial für Flüssigphasenchromatographie oder Gelpermeationschromatographie, als Polymerisate für optische Systeme, als Formteile, z.B. als Schicht für lichtempfindliche photographische Materialien, als wasser-und schmutzabweisende Schichten, als Filmmaterial zur Gastrennung, als Antifäulnisanstrich, zur Herstellung von Resists, als Materialien für Papier- und Textilbehandlung, als elastische Formteile z.B. Gummiartikel sowie als Klebstoffe.

Die Styrole (I) können auch als Synthesezwischenprodukte verwendet werden.

**Beispiele**

Verfahrensvariante A

1) <u>4-[2-(Heptafluorpropoxy-1.1.2-trifluoräthoxy]-benzaldehyd</u>

Zu seiner Mischung aus 12,2 g 4-Hydroxybenzaldehyd, 13,8 g Kaliumcarbonat und 200 ml Acetonitril wurden bei 20-30 °C unter Rühren 29,3 g Perfluorpropylvinyläther zugetropft. Nach Abklingen der exothermen Reaktion wurde filtriert, das Lösungsmittel bei Normaldruck abdestilliert und das Produkt unter verminderten Druck destilliert.

Ausbeute: 28,6 g (74 %) 4-[2-(Heptafluorpropoxy)-1.1.2-trifluoräthoxy]-benzaldehyd, Siedepunkt 79-80 °C/0,1 bar; farbloses Öl.

2)-6) In Tabelle 1 werden zu den Beispielen 2 bis 6 physikalische Eigenschaften verschiedener Haloalkoxyaldehyde der Formel

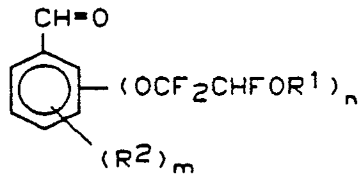

(VI)

aufgeführt. Die Herstellung erfolgte analog Beispiel 1.

Tabelle 1

| Nr. | $R^1$ | ($OCF_2CHFOR^1$)-Position a) | n | $R^2$ | m | Ausb. [%] | Sdp./Druck [°C]/[mbar] |
|---|---|---|---|---|---|---|---|
| 2 | $C_3F_7$- | 3 | 1 | H | 1 | 64 | 97-8/1,7 |
| 3 | $C_3F_7$- | 2 | 1 | H | 1 | 61 | 90 /1,8 |
| 4 | $CHF_2CF_2CF_2$- | 4 | 1 | H | 1 | 84 | 104 /0,5 |
| 5 | $CHF_2CF_2CF_2$- | 2 | 1 | H | 1 | 50 | 102 /0,6 |
| 6 | $C_3F_7$- | 2,3,4 | 3 | H | 1 | 66 | 134 /0,1 |

a) [-CH = O] in Position 1

7) 3-[2-(Heptafluorpropoxy)-1.1.2-trifluoräthoxy]-styrol

6,2 g Natriumamid wurden unter Stickstoff portionsweise bei 20-30 °C zu 53,4 g Methyltriphenylphosphoniumbromid in 230 ml trockenem Tetrahydrofuran unter Rühren zugesetzt und bei Raumtemperatur über Nacht gerührt.
Anschließend wurden bei minus 75 °C 44.7g 3-[2-(Heptafluorpropoxy)-1.1.2-trifluoräthoxy]-benzaldehyd zugetropft, der Ansatz auf Raumtemperatur erwärmt und noch 2 Stunden bei dieser Temperatur nachgerührt. Dann wurden 350 ml Hexan zugesetzt, auf minus 20 °C abgekühlt und das ausgefallene Triphenylphosphinoxid abfiltriert. Nach Destillation unter vermindertem Druck wurden 27,1 g (61%) 3-[2-(Heptafluorpropoxy)-1.1.2-trifluoräthoxy]-styrol erhalten; Siedepunkt 54 °C/0,1 mbar; farblose Flüssigkeit.

8)-9) In Tabelle 2 werden zu den Beispielen 8) und 9) physikalische Eigenschaften verschiedener Fluor-substituierter Styrolen der Formel

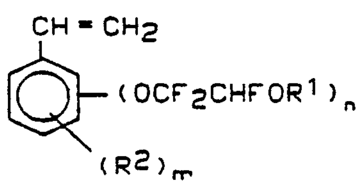

(I)

angeführt, die Herstellung erfolgte entsprechend Beispiel 7.

Tabelle 2

| Nr. | R' | ($OCF_2CHFOR'$)-Position b) | n | $R^2$ | m | Ausb. [%] | Sdp./Druck [°C]/[mbar] |
|---|---|---|---|---|---|---|---|
| 8 | $C_3F_7$- | 2 | 1 | H | 1 | 58 | 46 /0,1 |
| 9 | $C_3F_7$- | 2,3,4 | 3 | H | 1 | 60 | 135 /0,05 |

b) $-CH = CH_2$ in Position 1

Verfahrensvariante B

10) 4-[2-(Heptafluorpropoxy)-1.1.2-trifluoräthoxy]-styrol

168 g 50 % wäßrige Kalilauge wurden bei 20-30 °C unter Stickstoff zu 162 g 4-Acetoxystyrol und 0,1 g Hydrochinon in 2 l Acetonitril unter Rühren zugetropft.

Es wurden 24 Stunden nachgerührt. Bei 20 bis 30 °C wurden unter Rühren 319 g Perfluorpropylvinyläther zugetropft und 24 Stunden bei Raumtemperatur nachgerührt, die wäßrige Phase wurde abgetrennt, die organische Phase eingedampft und der Rückstand in Pentan aufgenommen. Diese Lösung wurde mit Wasser gewaschen, getrocknet und eingedampft. Eine Destillation des Rückstands unter verminderten Druck ergab 308 g (80 %) des Styrols in Form eines farblosen Öls, Siedepunkt 45 °C/0,05 mbar.

11) bis 13) Die in Tabelle 3 angeführten Styrole der Formel

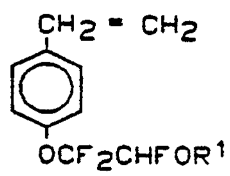

(I)

wurden analog Beispiel 10 hergestellt.

Tabelle 3

| Nr. | $R^1$ | Ausb. [%] | Sdp./Druck [°C]/[mbar] |
|---|---|---|---|
| 11 | $CHF_2CF_2CF_2$- | 77 | 56-63 0,1 |
| 12 | $C_3F_7O(CF_3)CFCF_2$- | 34 | 72 0,1 |
| 13 | $CF_3CF_2$- | 77 | 37 0,1 |

**Ansprüche**

1. Styrole der allgemeinen Formel

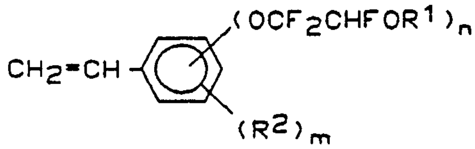

(I)

worin

$R^1$ teilweise oder vollständig halogeniertes $C_1$-$C_{10}$ Alkyl oder einen Rest der Formel

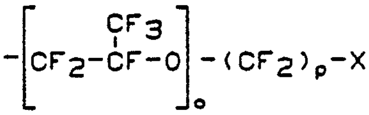

(II)

oder

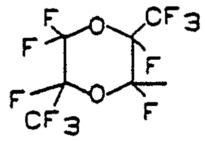

(III)

und $R^2$, Wasserstoff, Halogen oder eine teilweise oder vollständig halogenierte $C_1$-$C_8$ Alkyl-Gruppe, wobei die Alkylgruppen $R^1$ und $R^2$ gleich oder verschieden sind,
m ist als ganze Zahl 1, 2, 3 oder 4,
n ist als ganze Zahl 1, 2 oder 3,
o ist als ganze Zahl 1, 2, 3 oder 4,
p ist als ganze Zahl 1, 2, 3, 4, 5, 6, 7, 8, 9 oder 10
und X Wasserstoff oder Halogen bedeutet, mit der Maßgabe, daß die Summe aus m plus n nicht größer als fünf ist.

2) Styrole nach Anspruch 1, dadurch gekennzeichnet, daß Halogen Chlor oder Brom, vorzugsweise Fluor ist.

3) Verfahren zur Herstellung eines Styrols der allgemeinen Formel (I), dadurch gekennzeichnet, daß ein Hydroxyaldehyd der Formel

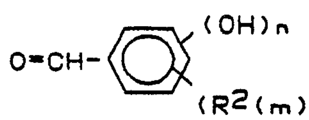

(IV)

in der $R^2$, m und n die obengenannte Bedeutung besitzen, in Gegenwart eines inerten, aprotischen Lösungsmittels ($C^1$) mit mindestens n Mol eines Vinyläthers der Formel

$CF_2 = CFOR^1$ (V)

worin $R^1$ die obengenannte Bedeutung besitzt, bei einer Temperatur von minus 100 bis + 100 °C, in Gegenwart einer Base $B^1$ zu einem Aldehyd allgemeinen Formel

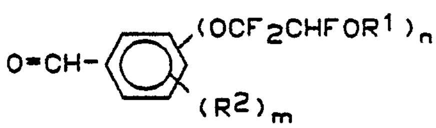

(VI)

worin $R^1$, $R^2$, m und n die obengenannte Bedeutung besitzen, umgesetzt wird und daß anschließend die Verbindung der Formel (VI) mit einem Methylentriarylphosphoniumhalogenid in Gegenwart einer Base $B^2$ in einem inerten wasserfreien Lösungsmittel ($C^2$) bei einer Temperatur von minus 100 bis 100 °C zu der

Verbindung (I) umgesetzt wird.

4. Verfahren nach Anspruch 3, dadurch gekennzeichnet, daß als Base $B^1$ Natrium- oder Kaliumcarbonat und als Base $B^2$ Natriumamid eingesetzt wird.

5) Verfahren zur Herstellung eines Styrols der allgemeinen Formel (I), dadurch gekennzeichnet, daß ein Acetoxystyrol der allgemeinen Formel

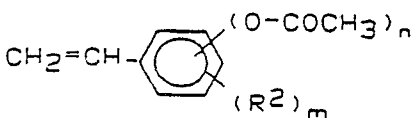

(VII),

worin $R^2$, m und n die obengenannte Bedeutung besitzen, zunächst mit einem Alkalihydroxid $B^3$ in einem inerten, aprotischen Lösungsmittel ($C^1$), und dann mit einem Vinyläther der Formel (V) umgesetzt wird, wobei die Temperatur der jeweiligen Verfahrensschritte minus 100 bis 100 °C beträgt.

6. Verfahren nach einem oder mehreren der Ansprüche 3 bis 5, dadurch gekennzeichnet, daß als inertes, aprotisches Lösungsmittel ($C^1$) Acetonitril und als inertes Lösungsmittel ($C^2$) Tetrahydrofuran eingesetzt wird.

7. Ausführungsform nach einem oder mehreren der Ansprüche 1 bis 6, dadurch gekennzeichnet, daß in der Formel (I) $R^1$ und $R^2$ jeweils eine teilweise oder vollständig halogenierte $C_1$-$C_3$-Alkyl-Gruppen ist.

8. Verwendung der Styrole der Formel (I) nach Anspruch 1 zur Herstellung von Polymeren

9. Verwendung nach Anspruch 8 als Trägermaterial für chromatographische Verfahren, als Material für optische Systeme, als Resists, als Formteile und Klebstoffe.